# EUROPEAN PATENT SPECIFICATION

(11) **EP 4 554 530 B1**
(45) Date of publication and mention of the grant of the patent: **09.09.2026**
(21) Application number: 23744672.9
(22) Date of filing: 13.07.2023
(51) Int. Cl.: A61F 5/445

(54) **OSTOMY APPLIANCE HAVING ADHESIVE LAYERS WITH DIFFERENT MOISTURE VAPOUR TRANSMISSION RATES**
OSTOMIEVORRICHTUNG MIT HAFTSCHICHTEN MIT VERSCHIEDENEN DAMPFDURCHLÄSSIGKEITEN
APPAREIL DE STOMIE AYANT DES COUCHES ADHÉSIVES AYANT DIFFÉRENTS TAUX DE TRANSMISSION DE VAPEUR D'HUMIDITÉ

(30) Priority: 14.07.2022 DK PA202270379
(43) Date of publication of application: 21.05.2025
(73) Proprietor: Coloplast A/S, 3050 Humlebaek (DK)
(72) Inventor: MOLZEN, Lars, 3450 Alleroed (DK); SUND, Anders Grove, 3450 Alleroed (DK); STROEBECH, Esben, 2970 Hoersholm (DK)
(86) International application number: PCT/DK2023/050187
(87) International publication number: WO 2024/012645

(56) References cited:
- WO-A1-2012/022352
- WO-A1-2019/174693
- WO-A1-2020/201689

## Description

### TECHNICAL FIELD

The present disclosure relates to an ostomy appliance and a method of manufacturing such ostomy appliance.

### BACKGROUND

Stomal output often contains body fluids and visceral contents that are aggressive to both the skin of a user and to ostomy devices. These have a detrimental effect on the efficiency and integrity of the adhesive materials that are applied to attach the ostomy device to the user's skin surface. For users in general, safe, reliable and efficient ostomy devices are evidently highly desirable.

However, a particularly major and persistent concern of a large population of ostomists continues to be failure of the base plate adhesive attaching the ostomy appliance to the user's skin surface, because such failure almost inevitably leads to embarrassing and stigmatising leakage incidents. Such incidents in turn are known from several user interviews to lead to a reduced quality-of-life feeling. Adhesive failure of the base plate adhesive can result from various reasons. Most often, a leakage incident is caused by stomal output entering between the proximal surface of the base plate and the user's skin, e.g. due to less-than-optimal attachment of the base plate to the skin arising from e.g. uneven skin surface or skin folds. This undesirable progression of stomal output "underneath" the adhesive leads to deterioration and/or weakening of the adhesive material carrying the weight and providing the seal of the ostomy appliance. Often, such failure happens surprisingly fast and is only detectable for the user once the failure has already become so severe that leakage occurs, requiring immediate change of the ostomy appliance and possibly also of the user's clothes.

In other instances, the primary factor of adhesive failure is simply a question of how much time has elapsed since the base plate of the ostomy appliance was first applied to the user's skin surface. In addition to the output from the stoma itself, the peristomal skin surface continuously secretes some moisture (e.g. sweat). To mitigate this, most often adhesives of base plates for ostomy devices include hydrocolloid materials which are capable of absorbing high levels of moisture, thereby stabilizing the polymer matrix of the adhesive material and prolonging the lifetime ("wear time") of the base plate. However, eventually the adhesion capability of the base plate no longer can support the force exerted on the base plate from the load of the output collecting bag, and the appliance must be replaced.

As there can be considerable differences in the severity and/or speed by which adhesive failure and potentially leakage occur, which differences at least to some extent are correlated to various factors including those presented above, a mere indication that failure or leakage is imminent, or that it has already occurred, fails to represent a reliable and satisfactory solution to the problem of avoiding sudden embarrassing and stigmatising leakage incidents in ostomy appliances. In other words, the users of ostomy appliances could greatly benefit from an appliance solution which provides them with better guidance and options regarding how and - not least - how quickly to react to beginning failure or leakage of the adhesive of the base plate of the appliance. More generally, ostomists and health care professionals alike would welcome improvements in ostomy devices to reduce or eliminate the occurrence of sudden leakage incidents.

WO 2019/174693 discloses a method for communicating a future operating state in an accessory device of an ostomy system.

### SUMMARY

On this background, it may be seen as an object of the present disclosure to provide an ostomy appliance comprising for example a sensor patch and/or a base plate for facilitating reliable and/or improved detection of risk of failure of an ostomy appliance and/or improved detection of risk of leakage. Another object of the present disclosure is to provide a method of manufacturing such an ostomy appliance.

One or more of these objects may be met by aspects of the present disclosure as described in the following.

A first aspect of this disclosure relates to an ostomy appliance for attachment to the skin surface of a user, wherein the ostomy appliance is adapted to form a stomal opening with a centre point, the stomal opening being configured to allow passage of output through the stomal opening and into an ostomy pouch attached to the ostomy appliance, the ostomy appliance comprising:
- a support layer with a distal surface and a proximal surface;
- a plurality of electrode paths for forming one or more sensors, the plurality of electrode paths being arranged on a first side of the support layer;
- a first adhesive layer arranged adjacent to, preferably on, the proximal surface of the support layer and being adapted for attachment to the skin surface of a user; and
- a second adhesive layer arranged on the first side of the support layer, the second adhesive layer having a first portion covering a first and a second electrode path of the plurality of electrode paths, the first and the second electrode paths being configured for forming a first sensor;
wherein the first portion of the second adhesive layer has a greater moisture vapour transmission rate (MVTR) than the first adhesive layer. The moisture vapour transmission rate (MVTR) of the first portion of the second adhesive layer may be at least two times, preferably at least three times, more preferably at least four times, greater than the moisture vapour transmission rate (MVTR) of the first adhesive layer.

The inventors have found that by providing adhesive layers with different moisture vapour transmission rates (MVTR), the layer interface of leakage initiation can be defined which may provide improved signals from the one or more sensors. For example, a leakage tends to initiate in a layer interface between the support layer and an adhesive layer with a MVTR which is greater than the MVTR of another adhesive layer interface.

Moisture vapour transmission rate (MVTR) is measured in grams per square meter (g/m²) over a 24-hour period using an inverted cup method.

A container or cup that was water and water vapour impermeable having an opening of Ø35 mm was used. 20 mL saline water (0.9% NaCl in demineralised water) was placed in the container and the opening was sealed with the test adhesive mounted on a highly permeable polyurethane (PU) backing film (BL9601 foil from Intellicoat). The container was placed into an electrically heated cabinet, and the container or cup was placed upside down, such that the water was in contact with the adhesive. The cabinet was maintained at 32°C. The film reference is used in all experiments to control for any variations in testing conditions.

The weight loss of the container was followed as a function of time. The weight loss was due to water transmitted through the adhesive and/or film. This difference was used to calculate the MVTR of the test adhesive film. MVTR was calculated as the weight loss per time divided by the area of the opening in the cup (g/m²/24h).

The MVTR of a material is a linear function of the thickness of the material. Thus, when reporting MVTR to characterize a material, it is important to inform the thickness of the material which MVTR was reported.

Finally, we noted that by using this method, we introduced an error by using a supporting PU film. Utilizing the fact that the adhesive/film laminate was a system of two resistances in series eliminated the error. When the film and the adhesive are homogeneous, the transmission rate may be expressed as: $1 / P \left(measured\right) = 1 / P \left(film\right) + 1 / P \left(adhesive\right) .$

Hence, by knowing the film permeability and thickness of the adhesive, it is possible to calculate the true permeability of the adhesive, P(adhesive), using the following expression: $P \left(adhesive\right) = d \left(adhesive\right) / 150 μm * 1 / \left(1/P\left(measured\right)-1/P\left(film\right)\right) ,$ where d(adhesive) was the actual measured thickness of the adhesive, and P(film) was the MVTR of the film without any adhesive on, and P(measured) was the actual measured MVTR.

Additionally or alternatively, the first adhesive layer may be made of a first composition and the first portion of the second adhesive layer may be made of a second composition. The first composition and/or the second composition may comprise one or more polyisobutenes and/or styrene-isoprene-styrene. The first composition and/or the second composition may comprise one or more hydrocolloids. The first composition and/or the second composition may comprise one or more water soluble or water swellable hydrocolloids. The first composition and/or the second composition may be a pressure sensitive adhesive composition suitable for medical purposes comprising a rubbery elastomeric base and one or more water soluble or water swellable hydrocolloids. The first composition and/or the second composition may comprise one or more polybutenes, one or more styrene copolymers, one or more hydrocolloids, or any combination thereof. The combination of the adhesive properties of the polybutenes and the absorbing properties of the hydrocolloids renders the first composition and/or the second composition suitable for use in ostomy appliances. For example, the styrene copolymer can be a styrene-butadiene-styrene block copolymer or a styrene-isoprene-styrene block copolymer. Preferably, one or more styrene-isoprene-styrene (SIS) block type copolymers are employed. The amount of styrene block-copolymer can be from 5% to 20% of the total adhesive composition. The butene component is suitably a conjugated butadiene polymer selected from polybutadiene, polyisoprene. The polybutenes are preferably present in an amount of from 35 to 50% of the total adhesive composition. Preferably, the polybutene is polyisobutylene (PIB). Suitable hydrocolloids for incorporation in the first composition and/or the second composition are selected from naturally occurring hydrocolloids, semisynthetic hydrocolloids, and synthetic hydrocolloids. The first composition and/or the second composition can comprise 20 to 60% hydrocolloids. A preferred hydrocolloid is carboxymethyl cellulose (CMC). Optionally, the first composition and/or the second composition can contain other components, such as fillers, tackifiers, plasticizers, and/or other additives. The first composition may preferably be different from the second composition. Preferably, the ingredients of the first composition and the second composition may be chosen so that the moisture vapour transmission rate (MVTR) of the second composition is greater than the moisture vapour transmission rate (MVTR) of the first composition, for example at least two times greater, preferably at least three times greater, or more preferably at least four times greater.

Additionally or alternatively, the first side of the support layer may be the distal surface of the support layer.

A leakage originating from the stoma tends to propagate the fastest through the adhesive layer with the greatest MVTR. By arranging the second adhesive layer on the distal surface of the support layer, the leakage may easily reach the second adhesive layer and cause minimal reduction of the adhesive performance of the first adhesive layer. Such arrangement may improve the sensor signal since the leakage initiates at an axial distance away from the skin surface of the user and radially away from the stoma and thus reduces sweat interference. Further, the high MVTR of the first portion of the second adhesive layer allows for the output/liquid to quickly propagate through the adhesive and reach the electrodes covered by said first portion of the second adhesive layer. Thereby, a signal indicative of leakage may be issued quickly, and potentially before the leakage causes breakdown of the adhesive performance of the first adhesive layer in contact with the skin.

Additionally or alternatively, the support layer may be moisture impermeable.

Providing the support layer as moisture impermeable advantageously electrically insulate sensor signals from moisture on the skin, and in particular moisture as absorbed by the first adhesive layer, and thus reduces sweat interference.

Additionally or alternatively, the support layer may comprise or consist essentially of a polymer film, preferably a polyurethane film.

Additionally or alternatively, the first sensor formed by the first and second electrode paths may be configured for allowing the provision of a signal indicative of a moisture level (e.g., an elevated moisture level) in the first portion of the second adhesive layer and/or indicative of liquid on a proximal surface of the first portion of the second adhesive layer, the signal preferably being indicative of a leak between the first portion of the second adhesive layer and the support layer. The signal (e.g., of the first sensor) may be provided by measuring the electrical resistance between one of the plurality of electrode paths (e.g., the first electrode path) and another of the plurality of electrode paths (e.g., the second electrode path).

Additionally or alternatively, the first portion of the second adhesive layer may be an inner portion arranged adjacent to the stomal opening. The inner portion may preferably be an inner ring portion that may extend circumferentially at least partly, but preferably entirely, around the stomal opening.

Additionally or alternatively, the second adhesive layer may comprise an outer portion. The inner portion may be arranged between the outer portion and the stomal opening. The outer portion may have a lower moisture vapour transmission rate (MVTR) than the inner ring portion. The outer portion may preferably be an outer ring portion that may extend circumferentially at least partly, but preferably entirely, around the stomal opening.

Additionally, the outer portion of the second adhesive layer may be formed of the same material (i.e. the same composition) as the first adhesive layer.

Such arrangements allow detection of a leakage initiated in the layer interface between the support layer and the firstinner portion while leakage and/or moisture propagation is slowed when the leakage reaches the outer portion.

Additionally or alternatively, the plurality of electrode paths may comprise a third and a fourth electrode path for forming a second sensor. The second sensor may be configured for allowing the provision of a signal indicative of a moisture level (e.g., an elevated moisture level) in the outer portion of the second adhesive layer and/or indicative of liquid on a proximal surface of the outer portion of the second adhesive layer, the signal preferably being indicative of a leak between the outer portion of the second adhesive layer and the support layer. Accordingly, the outer portion of the second adhesive layer may cover the third and fourth electrode path.

Additionally or alternatively, the support layer may comprise a ring portion and a neck portion. The ring portion may extend around the stomal opening. The neck portion may extend radially outwardly from the ring portion. The first adhesive layer may be arranged on the ring portion and the neck portion. The second adhesive layer may be arranged on the ring portion but not on the neck portion.

Additionally or alternatively, the ostomy appliance may comprise a first release liner arranged on and covering a proximal surface of the first adhesive layer.

Additionally or alternatively, the ostomy appliance may comprise a second release liner arranged on and covering a distal surface of the second adhesive layer.

Additionally or alternatively, the ostomy appliance may comprise a base plate. The base plate may include the support layer, the plurality of electrode paths, the first adhesive layer, and the second adhesive layer. In this embodiment, the ostomy appliance may thus be a one-piece ostomy appliance or a two-piece ostomy appliance. The one-piece ostomy appliance comprises, in addition to the base plate, an ostomy pouch fixedly attached to the base plate. The two-piece ostomy appliance comprises, in addition to the base plate, an ostomy pouch detachably attached to the base plate. For example, in the two-piece ostomy appliance, the base plate and the ostomy pouch may be releasably coupled e.g. with a mechanical and/or an adhesive coupling, e.g. to allow that a plurality of ostomy pouches can be utilized (exchanged) with one base plate. For example, the base plate may comprise a coupling ring for coupling an ostomy pouch to the base plate. Further, a two-piece ostomy appliance may facilitate correct application of the base plate to skin, e.g. to an improved user sight of the stomal region. The base plate may be configured for coupling to a user's stoma and/or skin surrounding the stoma, such as a peristomal skin area.

Additionally, the ostomy appliance may further comprise a backing layer. The second adhesive layer may be arranged between a proximal surface of the backing layer and a distal side of the support layer.

Alternatively, the ostomy appliance may comprise a sensor patch including the support layer, the plurality of electrode paths, the first adhesive layer, and the second adhesive layer. The sensor patch may further have a proximal side and a distal side. The distal side may be adapted for attachment to an adhesive surface of a base plate of the ostomy appliance. The proximal side may be adapted for attachment to a skin surface of a user. In this embodiment, the ostomy appliance may be a one-piece ostomy appliance or a two-piece ostomy appliance. The one-piece ostomy appliance comprises a base plate as well as an ostomy pouch fixedly attached to the base plate in addition to the sensor patch, which may be provided separately. The two-piece ostomy appliance comprises a base plate with a detachable ostomy pouch in addition to the sensor patch, which may be provided separately. For example, the base plate and the ostomy pouch may be releasably coupled e.g. with a mechanical and/or an adhesive coupling, e.g. to allow that a plurality of ostomy pouches can be utilized (exchanged) with one base plate. For example, the base plate may comprise a coupling ring for coupling an ostomy pouch to the base plate. Further, a two-piece ostomy appliance may facilitate correct application of the base plate to skin, e.g. to an improved user sight of the stomal region.

Additionally, the second adhesive layer may be adapted for attachment to a base plate.

A second aspect of this disclosure relates to a method of manufacturing an ostomy appliance according to the first aspect of this disclosure, the method comprising the steps of:
- providing a support layer with a distal surface and a proximal surface;
- arranging a plurality of electrode paths for forming one or more sensors on a first side of the support layer;
- arranging a first adhesive layer on the proximal surface of the support layer, the first adhesive layer being adapted for attachment to the skin surface of a user;
- arranging a second adhesive layer on the first side of the support layer, the second adhesive layer having a first portion covering the plurality of electrode paths;
wherein the first portion of the second adhesive layer has a greater moisture vapour transmission rate (MVTR) than the first adhesive layer. The moisture vapour transmission rate (MVTR) of the first portion of the second adhesive layer may be at least two times, preferably at least three times, more preferably at least four times, greater than the moisture vapour transmission rate (MVTR) of the first adhesive layer.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

Embodiments of this disclosure will be described in more detail in the following with regard to the accompanying figures. The figures show one way of implementing the present invention and are not to be construed as being limiting to other possible embodiments falling within the scope of the attached claim set.
Fig. 1 schematically illustrates an exploded view of an exemplary two-piece ostomy appliance.
Fig. 2 schematically illustrates an exploded view of an exemplary sensor patch of a two-piece ostomy appliance.
Fig. 3 schematically illustrates an exemplary electrode path configuration.
Fig. 4 schematically illustrates an exploded view of an exemplary sensor patch of an ostomy appliance.

### DETAILED DESCRIPTION

In the following detailed description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with respect to the orientation of the Figure(s) being described. Because components of embodiments can be positioned in a number of different orientations, the directional terminology is used for purposes of illustration and is in no way limiting. It is to be understood that other embodiments may be utilized, and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output", "waste(s)", and "fluids" interchangeably. A subject having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated or be implicit from the context that the "user" is not the "patient" him- or herself.

In the following, whenever referring to a proximal side of a device or part of a device, the referral is to the skin-facing side, when the ostomy appliance is worn by a user. Likewise, whenever referring to the distal side of a device or part of a device, the referral is to the side facing away from the skin, when the ostomy appliance is worn by a user. In other words, the proximal side is the side closest to the user when the appliance is fitted on a user, and the distal side is the opposite side - the side furthest away from the user when the appliance is fitted on the user.

The axial direction is defined as the direction of the stoma when the appliance is worn by a user. Thus, the axial direction is generally perpendicular to the skin or abdominal surface of the user.

The radial direction is defined as transverse to the axial direction that is transversely to the direction of the stoma, i.e. "across" the distal/proximal surface of the base plate. In some sentences, the words "inner" and "outer" may be used. These qualifiers should generally be perceived with respect to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do not provide the effect.

The use of the word "essentially" as a qualifier to certain structural and functional features or effects in this disclosure is used for emphasizing what is the most important focus of something or fact about something (i.e. a feature may have or fulfil a variety of effects, but when the disclosure discusses one effect as being "essentially" provided, this is the focus and the most important effect in relation to the disclosure).

Throughout the disclosure, the use of the terms "first", "second", "third", "fourth", "primary", "secondary", "tertiary" etc. does not imply any particular order or importance but is included merely to identify individual elements. Furthermore, the labelling of a first element does not imply the presence of a second element and vice versa.

Disclosed is an ostomy appliance which can either be a one-piece ostomy appliance or a two-piece ostomy appliance. The ostomy appliance comprises a base plate and an ostomy pouch (also referred to as an ostomy bag). The base plate is configured for coupling to a user's stoma and/or skin surrounding the stoma, such as a peristomal skin area. The ostomy appliance may be a colostomy appliance, an ileostomy appliance or a urostomy appliance. The ostomy appliance may be a two-piece ostomy appliance, i.e. the base plate and the ostomy pouch may be releasably coupled e.g. with a mechanical and/or an adhesive coupling, e.g. to allow that a plurality of ostomy pouches can be utilized (exchanged) with one base plate. For example, the base plate may comprise a coupling ring for coupling an ostomy pouch to the base plate. Further, a two-piece ostomy appliance may facilitate correct application of the base plate to skin, e.g. to an improved user sight of the stomal region. Alternatively, the ostomy appliance may be a one-piece ostomy appliance, i.e. the base plate and the ostomy pouch may be fixedly attached to each other. The one-piece or two-piece ostomy appliance may further include a separate sensor patch for attachment to the base plate. The sensor patch may facilitate detection of moisture propagation in an adhesive material as well as detection of a heightened risk of leakage. For example, the sensor patch may allow electronic measurements of performance of the base plate and/or to facilitate detection of increasing risks of leakage and/or to facilitate detection of decreasing adherence of the base plate to the skin of the user.

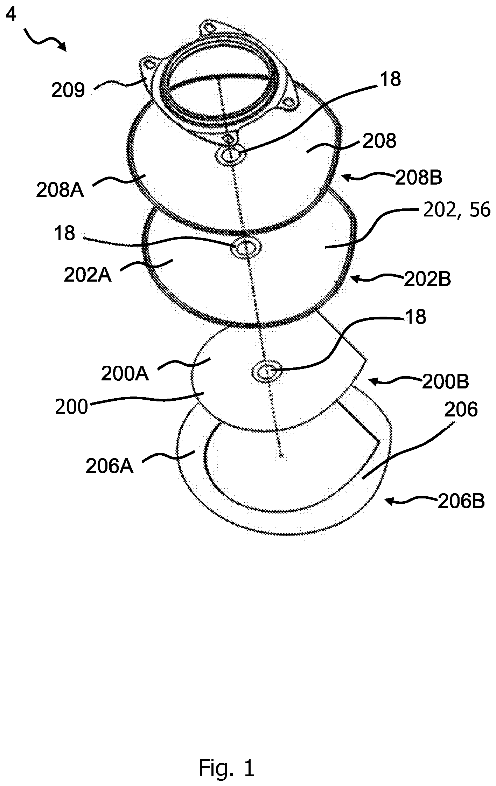

Fig. 1 schematically illustrates an exploded view of an exemplary two-piece ostomy appliance comprising a base plate 4. The base plate 4 comprises a first adhesive layer 200 having a distal surface 200A and a proximal surface 200B. During use, the proximal surface 200B of the first adhesive layer 200 adheres to the user's skin. The base plate 4 comprises a second adhesive layer 202 having a distal surface 202A and a proximal surface 202B. As illustrated, the second adhesive layer 202 spans a larger surface area than the first adhesive layer 200, such as to provide a rim of the proximal surface 202B of the second adhesive layer 202 surrounding the proximal surface 200B of the first adhesive layer 200.

The base plate 4 comprises a release liner 206, which may be peeled off by the user prior to applying the base plate 4 to the skin. The release liner 206 comprises a distal surface 206A and a proximal surface 206B. The distal surface 206A of the release liner 206 is covering the proximal surface of the first adhesive layer 200 and covering the proximal surface of the second adhesive layer 202 not covered by the first adhesive layer 200.

The base plate 4 comprises a backing layer 208. The backing layer 208 is a protective layer protecting the adhesive layers, such as the first adhesive layer 200 and/or the second adhesive layer 202 from external strains and stress during use. Furthermore, the backing layer 208 also covers the adhesive layers, such as the first adhesive layer 200 and/or the second adhesive layer 202, such that the adhesive layers 200, 202 do not adhere to clothes worn on top of the base plate 4. The backing layer 208 comprises a distal surface 208A and a proximal surface 208B. The distal surface 208A of the backing layer 208 is configured to face away from the skin of the user. The proximal surface 208B of the backing layer 208 is covering the second adhesive layer 202.

The base plate 4 forms part of a two-piece ostomy appliance, thus comprising a coupling ring 209 for coupling an ostomy pouch to the base plate 4, such as to a distal side of the base plate 4.

The base plate 4 comprises a stomal opening. The layers of the base plate 4, such as the first adhesive layer 200, the second adhesive layer 202 and the backing layer 208 as illustrated, may comprise stomal openings 18 for collectively forming the stomal opening of the base plate.

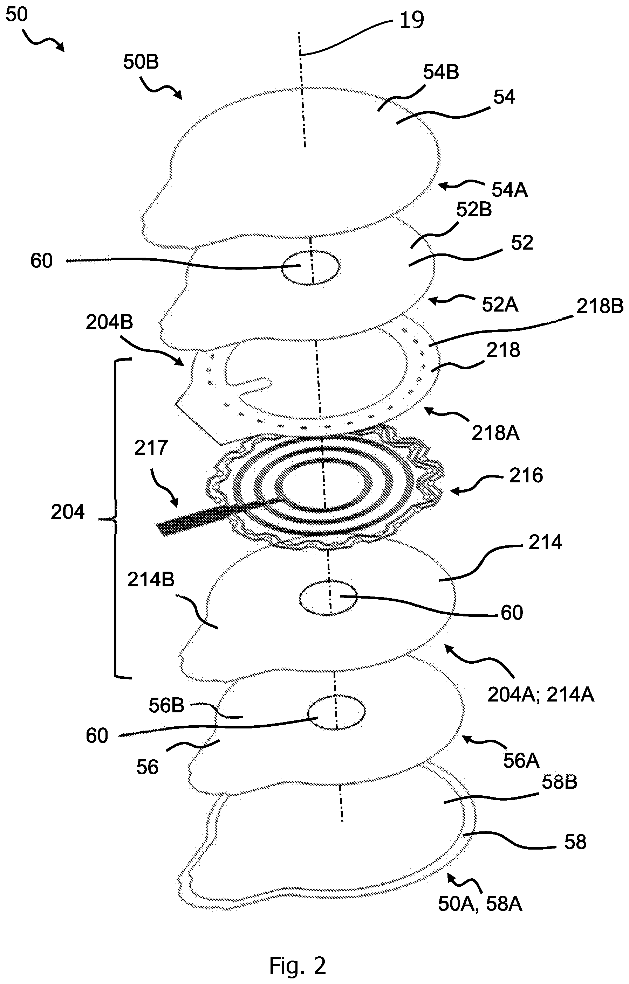

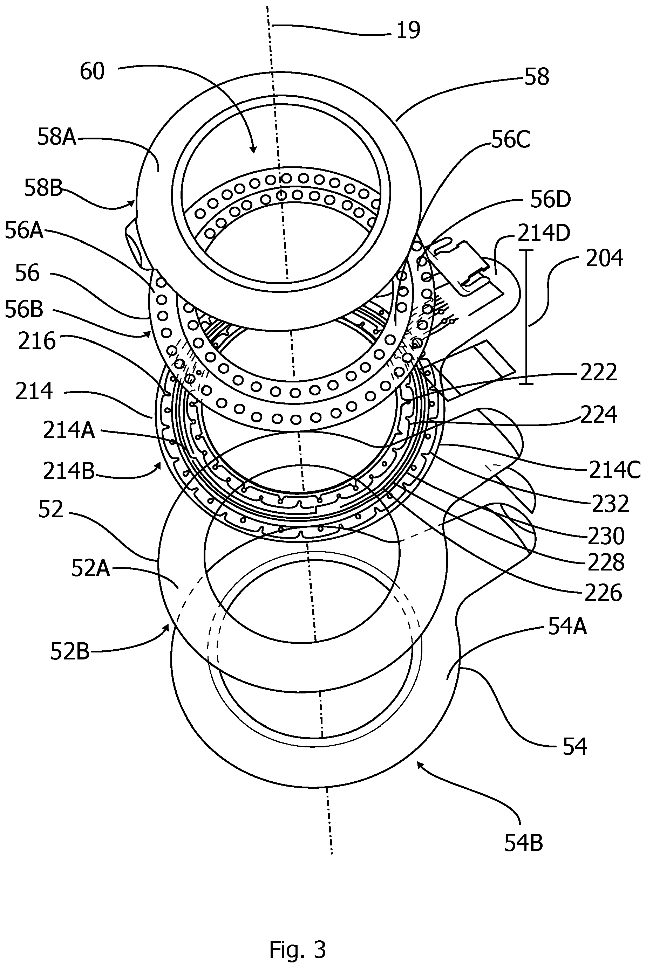

Figs. 2 and 3 both schematically illustrate an exploded view of exemplary two-piece ostomy appliances comprising a sensor patch 50, such as a sensor patch 50 being adapted for attachment to a base plate, such as the base plate 4 as illustrated in Fig. 1. The sensor patch 50 is configured to be positioned between the skin of the user and the proximal side of the base plate 4. For example, the sensor patch may be adapted for attachment to the first adhesive layer 200, such as the proximal surface 200B of the first adhesive layer 200, of the base plate 4. The sensor patch 50 is configured to be attached to the base plate such that the distal side 50A of the sensor patch 50 is attached to the proximal side of the base plate, such as to the proximal surface 200B of the first adhesive layer 200 of the base plate 4.

In Fig. 3, the sensor patch 50 comprises a sensor assembly 204 comprising a plurality of electrode paths 216 including for example a first electrode path 222, a second electrode path 224, a third electrode path 226, a fourth electrode path 228, a fifth electrode path 230, a sixth electrode path 232. Each electrode path 216 has respective connection parts 217 for connecting the plurality of electrode paths 216 to respective terminal elements of a monitor device. The sensor assembly 204 may form a sensor assembly layer.

In Figs. 2 and 3, the sensor assembly 204 has a distal side 204A and a proximal side 204B. The sensor assembly 204 comprises a support layer 214 with a distal surface 214A and a proximal surface 214B. The electrode paths 216 may be provided, such as formed, on the proximal surface 214B of the support layer 214, e.g. the electrode paths 216 may be positioned on the proximal surface 214B of the support layer 214 as shown in Fig. 2. Alternatively, the electrode paths may be provided, such as formed, on the distal surface 214A of the support layer 214, e.g. as shown in Fig. 3. The support layer 214 comprises a ring portion 214C extending around the entire stomal opening 60 and a neck portion 214D extending radially from the ring portion 214C.

As shown in Fig. 2 but not in Fig. 3, the electrode path assembly 204 may further comprise a masking element 218 having a distal surface 218A and a proximal surface 218B. The masking element 218 is configured to electrically insulate at least parts of the electrode paths 216 from adjacent layers, such as a first adhesive sensor layer 52 as shown in Fig. 2 or a second adhesive sensor layer 56 as shown in Fig. 3. The masking element 218 covers or overlaps with parts of the electrode paths 216 when seen in the axial direction.

Turning to both Figs. 2 and 3, the sensor patch 50 comprises the first adhesive sensor layer 52 with a proximal side 52B and a distal side 52A. The first adhesive sensor layer 52 is arranged on the proximal side 204B of the sensor assembly 204 and covering the neck portion 214D of the support layer 214. The proximal side 52B of the first adhesive sensor layer 52 is configured to adhere to the user's skin. Thus, after being applied to the base plate, the combined base plate and sensor patch 50 form an adhesive proximal surface configured to be applied to the skin surface of the user.

The sensor patch comprises a first sensor release liner 54. The first sensor release liner 54 may comprise a distal surface 54A and a proximal surface 54B. The first sensor release liner 54 may be arranged to protect the first adhesive sensor layer 52. The distal surface 54A of the first sensor release liner 54 is facing the proximal surface 52B of the first adhesive sensor layer 52. The first sensor release liner 54 is configured to be peeled off by the user prior to application of the base plate with the attached sensor patch to the skin. The first adhesive sensor layer 52 may be laid out on the distal side 54A of the first sensor release liner 54.

The sensor patch 50 comprises a second adhesive sensor layer 56, with a proximal side 56B and a distal side 56A. The second adhesive sensor layer 56 is arranged on the distal side 204A of the sensor assembly 204 but does not cover the neck portion 214D of the support layer 214. The proximal side 56B of the second adhesive sensor layer 52 is configured to adhere to the base plate, such as the proximal surface of the first adhesive layer of the base plate.

The sensor patch 50 comprises a stomal opening 60. The layers of the sensor patch 50, such as the first adhesive sensor layer 52, the support layer 214 and the second adhesive sensor layer 56, as illustrated, may comprise stomal openings 60 for collectively forming the stomal opening of the sensor patch 50. The stomal opening 60 of the sensor patch is configured to be aligned with the stomal opening 18 of the base plate, such as to collectively form the stomal opening of the combined base plate and sensor patch 50.

As shown in Figs. 2 and 3, the sensor patch 50 comprises a stomal opening 60 with a centre point 19 (here illustrated as by a centre line). In some exemplary sensor patches, the stomal opening 60 may be made by the user, i.e. the sensor patch may be manufactured and/or sold without the stomal opening 60, but with a region adapted to form the stomal opening 60 with the centre point 19.

As best seen in Fig. 3, the second adhesive sensor layer 56 comprises an inner ring portion 56C and an outer ring portion 56D both extending entirely around the stomal opening. The inner ring portion 56C is arranged adjacent to the stomal opening 60 and covers the first and second electrode paths 222, 224. The outer ring portion 56D extends on a radial outer side of the inner ring portion 56C and covers the third, fourth, fifth, and sixth electrode paths 226, 228, 230, 232. The first adhesive sensor layer 52 consists essentially of a first composition. The inner ring portion 56C of the second adhesive sensor layer 56 consists essentially of a second composition. The support layer 214 is advantageously moisture and liquid impermeable to avoid transfer of moisture, e.g. sweat, from the skin surface of the user to the inner ring portion 56C. The ingredients of the first and second compositions are chosen as discussed in the summary section so that the second composition has a greater moisture vapour transmission rate (MVTR) than the first composition. A leakage originating from the stoma tends to propagate the fastest through the adhesive layer with the greatest MVTR. Accordingly, the second composition wears out faster than the first composition and a leak will tend to initiate at the inner ring portion 56C of the second adhesive sensor layer 56. The outer ring portion 56D of the second adhesive sensor layer 56 is preferably made of the first composition to ensure similar wear time for the outer ring portion 56D and the first adhesive sensor layer 52. Such arrangement may improve the sensor signal since the leakage initiates at an axial distance away from the skin surface of the user and radially away from the stoma and thus reduces sweat interference. Further, the high MVTR of the inner ring portion 56C of the second adhesive sensor layer 56 allows for the output/liquid to quickly propagate through the second composition and reach the first and second electrode paths 222, 224 covered by the inner ring portion 56C of the second adhesive sensor layer 56. Thereby, a signal indicative of leakage may be issued quickly, and potentially before the leakage causes breakdown of the adhesive performance of the first adhesive sensor layer 52 in contact with the skin.

The exemplary sensor patch comprises a second sensor release liner 58. The second sensor release liner 58 may comprise a distal surface 58A and a proximal surface 58B. The second sensor release liner 58 may be arranged to protect the second adhesive sensor layer 56. The proximal surface 58B of the second sensor release liner 58 is facing the distal surface 56A of the second adhesive sensor layer 56. The second sensor release liner 58 is configured to be peeled off by the user prior to application of the sensor patch to the base plate. The second adhesive sensor layer 56 may be laid out on the proximal side 58B of the second sensor release liner 58.

Whereas the illustrated exemplary sensor patch 50 comprises a second adhesive sensor layer 56 arranged on the distal side 204A of the sensor assembly 204, it is envisioned that a first alternative ostomy appliance according to the disclosure may be provided with the second adhesive sensor layer 56 arranged on the proximal side 204B of the sensor assembly 204, for example arranged between the support layer 214 and the first adhesive sensor layer 52. Accordingly, the second sensor release liner 208 may be omitted. The distal surface of the support layer 214 may accordingly be configured for attachment to the adhesive surface of a base plate. The electrode paths 216 of such a first alternative sensor patch may then advantageously be arranged on the proximal side 214B of the support layer 214. In such a first alternative sensor patch, it is envisioned that the distal side 50A of the sensor patch 50 is configured to be attached to the first adhesive layer 200 of a base plate 4.

Furthermore, it is envisioned that a second alternative ostomy appliance according to the disclosure may be provided with the sensor patch 50 described in connection with Fig. 3 incorporated in a base plate 4. In such a second alternative ostomy appliance, the first adhesive layer 200 or the second adhesive layer 202 of the base plate as described in connection with Fig. 1 may further perform the functions of the second adhesive sensor layer 56, and the support layer 214 may be arranged between the first adhesive layer 200 and the second adhesive layer 202. The second adhesive layer 202 thus covers the plurality of electrodes 222, 224, 226, 228, 230, 232 being arranged on the distal side 214B of the support layer and the second adhesive layer 202 comprises the inner ring portion 56C and the outer ring portion 56D.

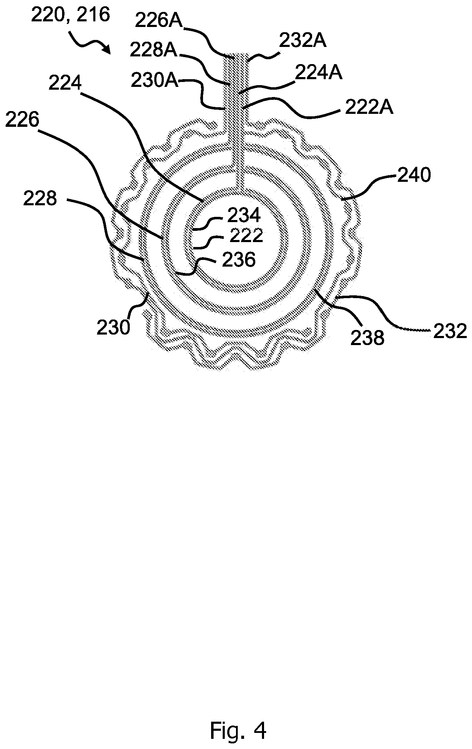

Fig. 4 schematically illustrates an exemplary electrode path configuration 220 of electrode paths 216 of an exemplary sensor assembly, such as the sensor assembly 204 as described with respect to Fig. 2. The electrode path configuration shown in Fig. 3 differs slightly from the configuration shown in Fig. 4 in that the two innermost electrode paths 222, 224 are arranged with discrete measurement points similar to the two outermost electrode paths 230, 232 shown in Fig. 4. Returning to Fig. 4, the plurality of electrode paths 216 comprises a first electrode path 222, a second electrode path 224, a third electrode path 226, a fourth electrode path 228, a fifth electrode path 230, and a sixth electrode path 232.

The first electrode path 222 comprises a first connection part 222A, and the second electrode path 224 comprises a second connection part 224A. The third electrode path 226 comprises a third connection part 226A. The fourth electrode path 228 comprises a fourth connection part 228A. The fifth electrode path 230 comprises a fifth connection part 230A. The sixth electrode path 232 comprises a sixth connection part 232A.

The first electrode path 222 may be a common ground electrode, also denoted a reference electrode, such as to form sensors with respect to the remaining electrode paths. The first electrode path 222 comprises a first electrode part 234 for forming a ground for the second electrode path 224. The first electrode path 222 comprises a second electrode part 236 for forming a ground for the third electrode path 226. The first electrode path 222 comprises a third electrode part 238 for forming a ground for the fourth electrode path 228. The first electrode path 222 comprises a fourth electrode part 240 for forming a ground for the fifth electrode path 230 and the sixth electrode path 232.
- 4: base plate
- 18: stomal opening
- 19: centre point
- 50: sensor patch
- 50A: distal side
- 50B: proximal side
- 52: first adhesive sensor layer
- 52A: distal side
- 52B: proximal side
- 54: first sensor release liner
- 54A: distal surface
- 54B: proximal surface
- 56: second adhesive sensor layer
- 56A: distal side
- 56B: proximal side
- 56C: inner ring portion
- 56D: outer ring portion
- 58: second sensor release liner
- 58A: distal surface
- 58B: proximal surface
- 60: stomal opening
- 200: first adhesive layer
- 200A: distal surface
- 200B: proximal surface
- 202: second adhesive layer
- 202A: distal surface
- 202B: proximal surface
- 204: sensor assembly
- 204A: distal side
- 204B: proximal side
- 206: release liner
- 206A: distal surface
- 206B: proximal surface
- 208: backing layer
- 208A: distal surface
- 208B: proximal surface
- 209: coupling ring
- 214: support layer
- 214A: distal surface
- 214B: proximal surface
- 214C: ring portion
- 214D: neck portion
- 216: electrode path
- 217: connection part
- 218: masking element
- 218A: distal surface
- 218B: proximal surface
- 220: electrode path configuration
- 222: first electrode path
- 222A: first connection part
- 224: second electrode path
- 224A: second connection part
- 226: third electrode path
- 226A: third connection part
- 228: fourth electrode path
- 228A: fourth connection part
- 230: fifth electrode path
- 230A: fifth connection part
- 232: sixth electrode path
- 232A: sixth connection part
- 234: first electrode part
- 236: second electrode part
- 238: third electrode part
- 240: fourth electrode part

## Claims

1. An ostomy appliance for attachment to the skin surface of a user, wherein the ostomy appliance is adapted to form a stomal opening (18, 60) with a centre point (19), the stomal opening (18, 60) being configured to allow passage of output through the stomal opening and into an ostomy pouch attached to the ostomy appliance, the ostomy appliance comprising:
- a support layer (214) with a distal surface (214A) and a proximal surface (214B);
- a plurality of electrode paths (216) for forming one or more sensors, the plurality of electrode paths being arranged on a first side of the support layer;
- a first adhesive layer (200) adapted for attachment to the skin surface of a user and arranged adjacent to the proximal surface (214B) of the support layer (214); and
- a second adhesive layer (202) arranged on the first side of the support layer (214), the second adhesive layer having a first portion covering a first (222) and a second electrode path (224) of the plurality of electrode paths, the first and the second electrode paths (222, 224) being configured for forming a first sensor;
**characterized in that** the first portion of the second adhesive layer (202) has a greater moisture vapour transmission rate than the first adhesive layer (200).

2. An ostomy appliance according to claim 1, wherein the first side of the support layer is the distal surface of the support layer.

3. An ostomy appliance according to claim 2, wherein the support layer is moisture impermeable.

4. An ostomy appliance according to any one of the previous claims, wherein the first sensor is configured for allowing the provision of a signal indicative of a moisture level in the first portion of the second adhesive layer and/or indicative of liquid on a proximal surface of the first portion of the second adhesive layer.

5. An ostomy appliance according to any one of the previous claims, wherein the first portion of the second adhesive layer is an inner portion arranged adjacent to the stomal opening.

6. An ostomy appliance according to claim 5, wherein the second adhesive layer comprises an outer portion, wherein the inner portion is arranged between the outer portion and the stomal opening, wherein the outer portion has a lower moisture vapour transmission rate than the inner portion.

7. An ostomy appliance according to claim 6, wherein the outer portion of the second adhesive layer is formed of the same material as the first adhesive layer.

8. An ostomy appliance according to any one of claims 6-7, wherein the plurality of electrode paths comprises a third (226) and a fourth electrode path (228) for forming a second sensor configured for allowing the provision of a signal indicative of a moisture level in the outer portion of the second adhesive layer and/or indicative of liquid on a proximal surface of the outer portion of the second adhesive layer.

9. An ostomy appliance according to any one of the previous claims, wherein the support layer (214) comprises a ring portion (214C) extending around the stomal opening, and a neck portion (214C) extending radially outwardly from the ring portion (214C), wherein the first adhesive layer (200) is arranged on the ring portion (214C) and the neck portion (214D), and wherein the second adhesive layer (200) is arranged on the ring portion (214C) but not on the neck portion (214D).

10. An ostomy appliance according to any one of the previous claims, further comprising a first release liner (206) arranged on and covering a proximal surface of the first adhesive layer and/or a second release liner arranged on and covering a distal surface of the second adhesive layer.

11. An ostomy appliance according to any one of the previous claims, wherein the ostomy appliance comprises a base plate (4), the base plate including the support layer, the plurality of electrode paths, the first adhesive layer, and the second adhesive layer.

12. An ostomy appliance according to claim 11 depending on at least claim 2, wherein the base plate further comprises a backing layer (208), wherein the second adhesive layer is arranged between a proximal surface (208B) of the backing layer and a distal side (214A) of the support layer (214).

13. An ostomy appliance according to any one of claims 1-10, wherein the ostomy appliance comprises a sensor patch (50) having a distal side (50A) and a proximal side (50B), the distal side (50A) being adapted for attachment to an adhesive surface of a base plate of the ostomy appliance, and the proximal side (50B) being adapted for attachment to a skin surface of a user, the sensor patch including the support layer, the plurality of electrode paths, the first adhesive layer and the second adhesive layer.

14. An ostomy appliance according to claim 13 depending on at least claim 2, wherein the second adhesive layer is adapted for attachment to a base plate.

15. A method of manufacturing an ostomy appliance according to any one of the previous claims, the method comprising the steps of:
- providing a support layer (214) with a distal surface (214A) and a proximal surface (214B);
- arranging a plurality of electrode paths (216) for forming one or more sensors on a first side of the support layer;
- arranging a first adhesive layer (200) on the proximal surface (214B) of the support layer (214), the first adhesive layer being adapted for attachment to the skin surface of a user; and
- arranging a second adhesive layer (202) on the first side of the support layer, the second adhesive layer having a first portion covering the plurality of electrode paths;
**characterized in that** the first portion of the second adhesive layer has a greater moisture vapour transmission rate (MVTR) than the first adhesive layer.

## Patentansprüche

1. Stomavorrichtung zur Befestigung an der Hautoberfläche eines Benutzers, wobei die Stomavorrichtung eingerichtet ist, um eine Stomaöffnung (18, 60) mit einem Mittelpunkt (19) zu bilden, wobei die Stomaöffnung (18, 60) dazu ausgelegt ist, Durchgang von Output durch die Stomaöffnung und in einen an der Stomavorrichtung befestigten Stomabeutel zu ermöglichen, wobei die Stomavorrichtung umfasst:
- eine Trägerschicht (214) mit einer distalen Oberfläche (214A) und einer proximalen Oberfläche (214B);
- eine Vielzahl von Elektrodenpfaden (216) zum Bilden eines oder mehrerer Sensoren, wobei die Vielzahl von Elektrodenpfaden auf der ersten Seite der Trägerschicht angeordnet ist;
- eine erste Klebeschicht (200), die zur Befestigung an der Hautoberfläche eines Benutzers eingerichtet und angrenzend an die proximale Oberfläche (214B) der Trägerschicht (214) angeordnet ist; und
- eine zweite Klebeschicht (202), die auf der ersten Seite der Trägerschicht (214) angeordnet ist, wobei die zweite Klebeschicht einen ersten Abschnitt aufweist, der einen ersten (222) und einen zweiten Elektrodenpfad (224) der Vielzahl von Elektrodenpfaden bedeckt, wobei der erste und der zweite Elektrodenpfad (222, 224) ausgelegt sind, um einen ersten Sensor zu bilden;
**dadurch gekennzeichnet, dass** der erste Abschnitt der zweiten Klebeschicht (202) eine größere Feuchtigkeitsdampfdurchlässigkeitsrate als die erste Klebeschicht (200) aufweist.

2. Stomavorrichtung nach Anspruch 1, wobei die erste Seite der Trägerschicht die distale Oberfläche der Trägerschicht ist.

3. Stomavorrichtung nach Anspruch 2, wobei die Trägerschicht feuchtigkeitsundurchlässig ist.

4. Stomavorrichtung nach einem der vorhergehenden Ansprüche, wobei der erste Sensor dazu ausgelegt ist, die Bereitstellung eines Signals zu ermöglichen, das ein Feuchtigkeitsniveau in dem ersten Abschnitt der zweiten Klebeschicht angibt und/oder Flüssigkeit auf einer proximalen Oberfläche des ersten Abschnitts der zweiten Klebeschicht angibt.

5. Stomavorrichtung nach einem der vorhergehenden Ansprüche, wobei der erste Abschnitt der zweiten Klebeschicht ein innerer Abschnitt ist, der angrenzend an die Stomaöffnung angeordnet ist.

6. Stomavorrichtung nach Anspruch 5, wobei die zweite Klebeschicht einen äußeren Abschnitt umfasst, wobei der innere Abschnitt zwischen dem äußeren Abschnitt und der Stomaöffnung angeordnet ist, wobei der äußere Abschnitt eine geringere Feuchtigkeitsdampfdurchlässigkeit als der innere Abschnitt aufweist.

7. Stomavorrichtung nach Anspruch 6, wobei der äußere Abschnitt der zweiten Klebeschicht aus dem gleichen Material wie die erste Klebeschicht gebildet ist.

8. Stomavorrichtung nach einem der Ansprüche 6-7, wobei die Vielzahl von Elektrodenpfaden einen dritten (226) und einen vierten Elektrodenpfad (228) zum Bilden eines zweiten Sensors umfasst, der dazu ausgelegt ist, die Bereitstellung eines Signals zu ermöglichen, das ein Feuchtigkeitsniveau in dem äußeren Abschnitt der zweiten Klebeschicht angibt und/oder Flüssigkeit auf einer proximalen Oberfläche des äußeren Abschnitts der zweiten Klebeschicht angibt.

9. Stomavorrichtung nach einem der vorhergehenden Ansprüche, wobei die Trägerschicht (214) einen Ringabschnitt (214C), der sich um die Stomaöffnung erstreckt, und einen Halsabschnitt (214C), der sich radial von dem Ringabschnitt (214C) nach außen erstreckt, umfasst, wobei die erste Klebeschicht (200) auf dem Ringabschnitt (214C) und dem Halsabschnitt (214D) angeordnet ist, und wobei die zweite Klebeschicht (200) auf dem Ringabschnitt (214C), jedoch nicht auf dem Halsabschnitt (214D) angeordnet ist.

10. Stomavorrichtung nach einem der vorhergehenden Ansprüche, ferner umfassend einen ersten Trennliner (206), der auf einer proximalen Oberfläche der ersten Klebeschicht angeordnet ist und diese bedeckt, und/oder einen zweiten Trennliner, der auf einer distalen Oberfläche der zweiten Klebeschicht angeordnet ist und diese bedeckt.

11. Stomavorrichtung nach einem der vorhergehenden Ansprüche, wobei die Stomavorrichtung eine Basisplatte (4) umfasst, wobei die Basisplatte die Trägerschicht, die Vielzahl von Elektrodenpfaden, die erste Klebeschicht und die zweite Klebeschicht einschließt.

12. Stomavorrichtung nach Anspruch 11 in Abhängigkeit von mindestens Anspruch 2, wobei die Basisplatte ferner eine Unterlagenschicht (208) umfasst, wobei die zweite Klebeschicht zwischen einer proximalen Oberfläche (208B) der Unterlagenschicht und einer distalen Seite (214A) der Trägerschicht (214) angeordnet ist.

13. Stomavorrichtung nach einem der Ansprüche 1-10, wobei die Stomavorrichtung ein Sensorpflaster (50) mit einer distalen Seite (50A) und einer proximalen Seite (50B) umfasst, wobei die distale Seite (50A) zur Befestigung an einer Klebeoberfläche einer Basisplatte der Stomavorrichtung eingerichtet ist, und wobei die proximale Seite (50B) zur Befestigung an einer Hautoberfläche eines Benutzers eingerichtet ist, wobei das Sensorpflaster die Trägerschicht, die Vielzahl von Elektrodenpfaden, die erste Klebeschicht und die zweite Klebeschicht einschließt.

14. Stomavorrichtung nach Anspruch 13 in Abhängigkeit von mindestens Anspruch 2, wobei die zweite Klebeschicht zur Befestigung an einer Basisplatte eingerichtet ist.

15. Verfahren zum Fertigen einer Stomavorrichtung nach einem der vorhergehenden Ansprüche, wobei das Verfahren die folgenden Schritte umfasst:
- Bereitstellen einer Trägerschicht (214) mit einer distalen Oberfläche (214A) und einer proximalen Oberfläche (214B);
- Anordnen einer Vielzahl von Elektrodenpfaden (216) zum Bilden eines oder mehrerer Sensoren auf einer ersten Seite der Trägerschicht;
- Anordnen einer ersten Klebeschicht (200) auf der proximalen Oberfläche (214B) der Trägerschicht (214), wobei die erste Klebeschicht zur Befestigung an der Hautoberfläche eines Benutzers eingerichtet ist; und
- Anordnen einer zweiten Klebeschicht (202) auf der ersten Seite der Trägerschicht, wobei die zweite Klebeschicht einen ersten Abschnitt aufweist, der die Vielzahl von Elektrodenpfaden bedeckt;
**dadurch gekennzeichnet, dass** der erste Abschnitt der zweiten Klebeschicht eine größere Feuchtigkeitsdampfdurchlässigkeitsrate (MVTR) als die erste Klebeschicht aufweist.

## Revendications

1. Appareil de stomie destiné à être fixé à la surface cutanée d'un utilisateur, l'appareil de stomie étant adapté pour former une ouverture stomale (18, 60) avec un point central (19), l'ouverture stomale (18, 60) étant configurée pour permettre le passage de la sortie à travers l'ouverture stomale et dans une poche de stomie fixée à l'appareil de stomie, l'appareil de stomie comprenant :
- une couche de support (214) avec une surface distale (214A) et une surface proximale (214B) ;
- une pluralité de chemins d'électrode (216) pour former un ou plusieurs capteurs, la pluralité de chemins d'électrode étant agencée sur un premier côté de la couche de support ; et
- une première couche adhésive (200) adaptée pour être fixée sur la surface cutanée d'un utilisateur et disposée de manière adjacente à la surface proximale (214B) de la couche de support (214) ; et
- une seconde couche adhésive (202) disposée sur le premier côté de la couche de support (214), la seconde couche adhésive présentant une première partie recouvrant un premier (222) et un deuxième chemin d'électrode (224) de la pluralité de chemins d'électrode, les premier et deuxième chemins d'électrode (222, 224) étant configurés pour former un premier capteur ;
**caractérisé en ce que** la première partie de la seconde couche adhésive (202) présente un taux de transmission de vapeur d'eau plus élevé que la première couche adhésive (200).

2. Appareil de stomie selon la revendication 1, dans lequel le premier côté de la couche de support est la surface distale de la couche de support.

3. Appareil de stomie selon la revendication 2, dans lequel la couche de support est imperméable à l'humidité.

4. Appareil de stomie selon l'une quelconque des revendications précédentes, dans lequel le premier capteur est configuré pour permettre la fourniture d'un signal indiquant un niveau d'humidité dans la première partie de la seconde couche adhésive et/ou indiquant un liquide sur une surface proximale de la première partie de la seconde couche adhésive.

5. Appareil de stomie selon l'une quelconque des revendications précédentes, dans lequel la première partie de la seconde couche adhésive est une partie interne disposée adjacente à l'ouverture stomale.

6. Appareil de stomie selon la revendication 5, dans lequel la seconde couche adhésive comprend une partie externe, dans lequel la partie interne est agencée entre la partie externe et l'ouverture stomale, dans lequel la partie externe présente un taux de transmission de vapeur d'eau inférieur à celui de la partie interne.

7. Appareil de stomie selon la revendication 6, dans lequel la partie externe de la seconde couche adhésive est formée du même matériau que la première couche adhésive.

8. Appareil de stomie selon l'une quelconque des revendications 6 à 7, dans lequel la pluralité de chemins d'électrode comprend un troisième (226) et un quatrième chemin d'électrode (228) pour former un second capteur configuré pour permettre la fourniture d'un signal indiquant un niveau d'humidité dans la partie externe de la seconde couche adhésive et/ou indiquant un liquide sur une surface proximale de la partie externe de la seconde couche adhésive.

9. Appareil de stomie selon l'une quelconque des revendications précédentes, dans lequel la couche de support (214) comprend une partie annulaire (214C) s'étendant autour de l'ouverture stomale, et une partie col (214C) s'étendant radialement vers l'extérieur à partir de la partie annulaire (214C), dans lequel la première couche adhésive (200) est agencée sur la partie annulaire (214C) et la partie col (214D), et dans lequel la seconde couche adhésive (200) est agencée sur la partie annulaire (214C) mais pas sur la partie col (214D).

10. Appareil de stomie selon l'une quelconque des revendications précédentes, comprenant en outre une première doublure détachable (206) agencée sur une surface proximale de la première couche adhésive et recouvrant celle-ci, et/ou une seconde doublure détachable agencée sur une surface distale de la seconde couche adhésive et recouvrant celle-ci.

11. Appareil de stomie selon l'une quelconque des revendications précédentes, dans lequel l'appareil de stomie comprend une plaque de base (4), la plaque de base comportant la couche de support, la pluralité de chemins d'électrode, la première couche adhésive et la seconde couche adhésive.

12. Appareil de stomie selon la revendication 11 dépendant d'au moins la revendication 2, dans lequel la plaque de base comprend en outre une couche de support (208), dans lequel la seconde couche adhésive est agencée entre une surface proximale (208B) de la couche de support et un côté distal (214A) de la couche de support (214).

13. Appareil de stomie selon l'une quelconque des revendications 1 à 10, l'appareil de stomie comprenant un timbre de détection (50) ayant un côté distal (50A) et un côté proximal (50B), le côté distal (50A) étant adapté pour être fixé à une surface adhésive d'une plaque de base de l'appareil de stomie, et le côté proximal (50B) étant adapté pour être fixé à une surface cutanée d'un utilisateur, le timbre de détection comportant la couche de support, la pluralité de chemins d'électrode, la première couche adhésive et la seconde couche adhésive.

14. Appareil de stomie selon la revendication 13 dépendant au moins de la revendication 2, dans lequel la seconde couche adhésive est adaptée pour être fixée à une plaque de base.

15. Procédé de fabrication d'un appareil de stomie selon l'une quelconque des revendications précédentes, le procédé comprenant les étapes suivantes :
- la fourniture d'une couche de support (214) ayant une surface distale (214A) et une surface proximale (214B) ;
- l'agencement d'une pluralité de chemins d'électrode (216) pour former un ou plusieurs capteurs sur un premier côté de la couche de support ; et
- l'agencement d'une première couche adhésive (200) sur la surface proximale (214B) de la couche de support (214), la première couche adhésive étant adaptée pour être fixée sur la surface cutanée d'un utilisateur ; et
- l'agencement d'une seconde couche adhésive (202) sur le premier côté de la couche de support, la seconde couche adhésive présentant une première partie recouvrant la pluralité de chemins d'électrode ;
**caractérisé en ce que** la première partie de la seconde couche adhésive présente un taux de transmission de vapeur d'eau (MVTR) plus élevé que la première couche adhésive.
